(19) Europäisches Patentamt
European Patent Office
Office européen des brevets

(11) **EP 4 317 933 A1**

(12) **EUROPEAN PATENT APPLICATION**
published in accordance with Art. 153(4) EPC

(43) Date of publication:
**07.02.2024 Bulletin 2024/06**

(21) Application number: **22781319.3**

(22) Date of filing: **31.03.2022**

(51) International Patent Classification (IPC):
*G01M 3/20* (2006.01)     *H01M 8/00* (2016.01)
*H01M 8/04* (2016.01)     *H01M 8/04313* (2016.01)
*H01M 8/0438* (2016.01)     *H01M 8/0444* (2016.01)
*H01M 8/04492* (2016.01)     *H01M 8/10* (2016.01)
*H01M 8/12* (2016.01)     *G01M 17/007* (2006.01)

(52) Cooperative Patent Classification (CPC):
**G01M 3/20; G01M 17/007; H01M 8/00; H01M 8/04;
H01M 8/04313; H01M 8/0438; H01M 8/0444;
H01M 8/04492; H01M 8/10; H01M 8/12;
Y02E 60/50**

(86) International application number:
**PCT/JP2022/016943**

(87) International publication number:
**WO 2022/211111 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.04.2021   JP 2021063172
13.05.2021   JP 2021081988**

(71) Applicant: HORIBA, Ltd.
**Kyoto-shi, Kyoto 601-8510 (JP)**

(72) Inventors:
• **OIDA, Takuji**
  **Kyoto-shi, Kyoto 601-8510 (JP)**
• **NAKATANI, Shigeru**
  **Kyoto-shi, Kyoto 601-8510 (JP)**
• **AOYAMA, Junichi**
  **Kyoto-shi, Kyoto 601-8510 (JP)**
• **YOKOTA, Yoshihiro**
  **Kyoto-shi, Kyoto 601-8510 (JP)**

(74) Representative: **Müller Hoffmann & Partner
Patentanwälte mbB
St.-Martin-Strasse 58
81541 München (DE)**

(54) **SYSTEM FOR MEASURING AMOUNT OF CONSUMED HYDROGEN**

(57)   There is provided a hydrogen consumption quantity measurement system that, even in a case in which there is a possibility that leakage hydrogen is contained in exhaust gas from a fuel cell or a hydrogen engine, makes it possible to accurately determine a total quantity of hydrogen consumption of the fuel cell without having to modify a vehicle or the like. This hydrogen consumption quantity measurement system measures a hydrogen consumption quantity in a test body, which is formed by a moving body or portion thereof that includes a hydrogen reactor that causes hydrogen to undergo a chemical reaction, and that utilizes energy obtained from this chemical reaction, and includes an oxygen concentration sensor that measures a concentration of oxygen contained in exhaust gas from the test body, an oxidation catalyst that is disposed between the hydrogen reactor and the oxygen concentration sensor and that oxidizes hydrogen contained in the exhaust gas using oxygen contained in that exhaust gas, and a hydrogen consumption quantity calculation unit that, based on the oxygen concentration measured by the oxygen concentration sensor, calculates a hydrogen consumption quantity.

FIG.1

**Description**

[Technical Field]

The present invention relates to a system for measuring a quantity of hydrogen that is consumed in a fuel cell or the like.

[Technical Background]

As is described in Non-Patent Document 1, a mass method, a pressure method, and a flow rate method and the like, as prescribed in ISO 23828, are used as fuel consumption test methods for fuel cell vehicles. However, each of these methods involves supplying hydrogen from outside the vehicle, and this requires modifying the vehicle so as to enable a hydrogen supply line for supplying hydrogen from outside the vehicle to be interposed on the hydrogen supply line of the fuel cell vehicle. On the other hand, both domestically and overseas, the importance of completed vehicle inspections is increasing and there is a demand for the establishment of a fuel consumption test method that does not require the vehicle to be modified.

For this reason, an oxygen balance method such as that described in Non-Patent Documents 1 and 2 has been proposed for a system to measure the hydrogen consumption quantity of a fuel cell that does not require any vehicle modification.

In this oxygen balance method, the quantity of hydrogen consumption in a fuel cell is calculated by using an oxygen concentration sensor to measure an amount of change between a concentration of oxygen that is taken in by a fuel cell in order for the hydrogen to undergo an electrochemical reaction, and a concentration of oxygen that is contained in exhaust gas expelled from the fuel cell after this reaction has ended.

[Documents of the Prior Art]

[Non-Patent Documents]

[Non-Patent Document 1] "Development of a Fuel Consumption Measuring Method without Vehicle Modification - Oxygen Balance Method using a Constant Volume Sampler", Masaru YANO, Takuya SHIMOSAKA, Shigeyuki ISHIDOYA, JARI Research Journal (20200401)

[Non-Patent Document 2] "Development of Fuel-Consumption Measuring Method for FCV without Vehicle Modification - Experiment Verification of Oxygen-Balance Method", Masaru YANO, Eiji KURODA, JARI Research Journal Vol. 30 No. 7 (2008.07)

[Disclosure of the Invention]

[Problems to be Solved by the Invention]

During an electrochemical reaction, a fuel cell behaves in such a way that the concentration of the hydrogen stored on an anode side decreases due to the hydrogen being consumed as a result of electricity being generated in the fuel cell. Because of this, in the fuel cell, in order to maintain the hydrogen concentration on the anode side at a fixed level, processing to release all of the gases remaining on the anode side through a discharge port, and take new hydrogen into the fuel cell is performed periodically. This is known as purging. In addition, there is also a phenomenon known as cross-leaking in which hydrogen performs membrane permeation through an anode wall surface and travels towards a cathode side so that the hydrogen is released from the fuel cell in an unreacted state.

In order to determine the quantity of hydrogen consumption in a fuel cell, in addition to the quantity of hydrogen consumed directly by the electrochemical reaction, there is a need to calculate a quantity of hydrogen consumption that also includes hydrogen leakage quantities occurring through purging and cross-leaking and the like.

However, using the aforementioned oxygen balance methods it is not possible to calculate a quantity of hydrogen consumption that includes hydrogen leakage quantities caused by the aforementioned purging and the like simply by performing an oxygen concentration measurement using an oxygen concentration sensor. For this reason, a means for directly measuring a hydrogen leakage quantity using a separately provided hydrogen concentration sensor is described in Non-Patent Documents 1 and 2 as a method for measuring a hydrogen consumption quantity that also includes hydrogen leakage quantities using an oxygen balance method.

In Non-Patent Document 1, as is described above, the hydrogen consumption quantity is measured using an oxygen balance method after firstly providing a separate hydrogen concentration sensor, however, Non-Patent Document 1 shows that it is necessary to further advance the discovery and improvement of causes of errors in order to go beyond targeted measurement errors.

Moreover, when measuring a hydrogen consumption quantity using a hydrogen concentration sensor, in a case in which it is necessary to improve measurement accuracy, it is conceivable that a high-precision hydrogen concentration sensor will be required.

In Non-Patent Document 2, an oxygen concentration sensor that uses a paramagnetic method (hereinafter, this may be referred to as a paramagnetic oxygen concentration sensor) is described as an example of an oxygen concentration sensor used in an oxygen balance method. A paramagnetic method is a method in which, using the magnetic characteristics of oxygen (more specifically, the magnetic susceptibility thereof), the magnetic susceptibility of a subject gas that has been compared with an auxiliary gas (which is a type of reference gas) whose oxygen concentration is known is converted into an oxygen concentration.

It is a principal object of the present invention to provide a measurement system that, even in a case in which there is a possibility that leakage hydrogen originating from purges and the like is contained in exhaust gas from a hydrogen reactor such as a fuel cell or the like, makes it possible to accurately calculate a total quantity of hydrogen consumption of the hydrogen reactor without having to provide a separate hydrogen concentration sensor or modify the vehicle.

[Means for Solving the Problem]

In other words, a hydrogen consumption quantity measurement system according to the present invention is a hydrogen consumption quantity measurement system that measures a hydrogen consumption quantity in a test body, which is formed by a moving body or portion thereof that includes a hydrogen reactor that causes hydrogen to undergo a chemical reaction, and that utilizes energy obtained from this chemical reaction, and is characterized in being provided with an oxygen concentration sensor that measures a concentration of oxygen contained in exhaust gas from the test body, an oxidation catalyst that is disposed between the hydrogen reactor and the oxygen concentration sensor and that oxidizes hydrogen contained in the exhaust gas using oxygen contained in that exhaust gas, and a hydrogen consumption quantity calculation unit that, based on the oxygen concentration measured by the oxygen concentration sensor, calculates a hydrogen consumption quantity.

According to this type of hydrogen consumption quantity measurement system, because there is provided an oxidation catalyst that oxidizes hydrogen contained in exhaust gas using oxygen contained that same exhaust gas, even in a case in which leakage hydrogen is contained in the exhaust gas, it is possible to oxidize this leakage hydrogen using oxygen contained in the exhaust gas. As a result, without having to measure the quantity of hydrogen leakage using a separate hydrogen concentration sensor in order to correct any errors in the oxygen balance method, as is the case conventionally, it is possible to accurately calculate the overall quantity of hydrogen consumed by a fuel cell including leakage hydrogen.

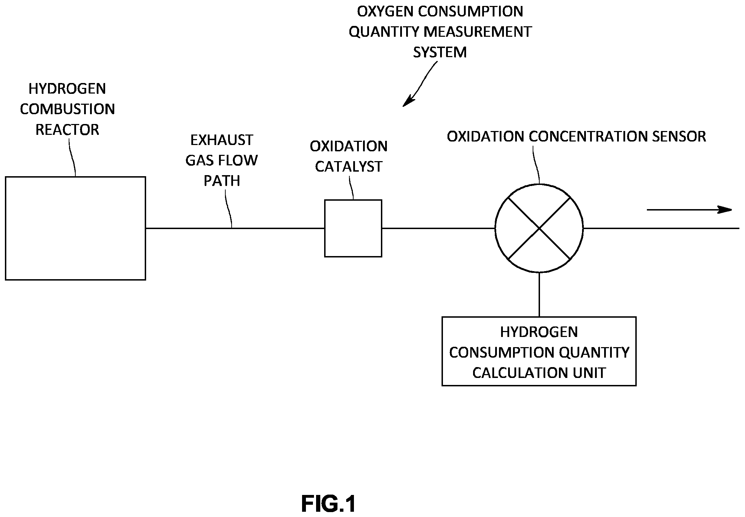

According to a hydrogen consumption quantity measurement system having the above-described structure such as that shown, for example, in FIG. 1, it is possible to calculate a hydrogen consumption quantity in a hydrogen reactor in the following manner.

Firstly, in the structure shown in FIG. 1, ambient air is allowed to enter an exhaust gas flow path by removing a pipe between the oxidation catalyst and the oxygen concentration sensor so as to open these up, and a concentration of oxygen in the air (A) is measured by the oxygen concentration sensor.

Next, the oxidation catalyst is removed via the exhaust gas flow path, for example, from the exhaust gas flow path of the hydrogen consumption quantity measurement system once this has been restored to the structure shown in FIG. 1. The exhaust gas is then guided to the oxygen concentration sensor from the hydrogen reactor without passing through the oxidation catalyst, and a concentration of oxygen in the exhaust gas (B) is measured by the oxygen concentration sensor.

Furthermore, in the hydrogen consumption quantity measurement system that has again been restored to the structure shown in FIG. 1, a concentration of oxygen in the exhaust gas (C) that has been guided to the oxygen concentration sensor after having been passed through the oxidation catalyst is measured.

By applying the oxygen concentration (A), the oxygen concentration (B), and the oxygen concentration (C) that have been calculated via the methods described above to the following calculation equations, it is possible to calculate the quantity of hydrogen consumed as fuel in the fuel cell that does not include leakage hydrogen, a total quantity of hydrogen consumption that includes leakage hydrogen, and an oxygen concentration difference corresponding to the leakage hydrogen quantity.

(Oxygen concentration corresponding to a hydrogen quantity that does not include

leakage hydrogen) $= A - B$

(Oxygen concentration corresponding to a total hydrogen quantity that does include

leakage hydrogen) $= A - C$

(Oxygen concentration corresponding to leakage hydrogen quantity)

$$= (A - C) - (A - B)$$

As is described above, by using the oxidation catalyst it is possible to cause leakage hydrogen present in a sample gas to undergo an oxidation reaction so as to be converted into water, and by ascertaining how much the quantity of oxygen has decreased after the sample gas has passed through the oxidation catalyst compared to before it passed through the oxidation catalyst (i.e., the quantity thereof that has undergone an oxidation reaction with leakage hydrogen), it is possible to determine the quantity of leakage hydrogen.

In an oxygen balance method, because it is necessary to measure minute changes in an oxygen concentration, in a case in which a paramagnetic oxygen concentration sensor, for example, is used as the oxygen concentration sensor, a standard gas is normally used for the auxiliary gas, however, even in a case in which ambient air or the like is used as the auxiliary gas, by determining a differential between the concentration of oxygen in the exhaust gas and the concentration of oxygen in the auxiliary gas and then further determining the flow rate of the gas that is flowing into the oxygen concentration sensor, if these are multiplied by each other it is possible to accurately determine the quantity of oxygen consumed in the hydrogen reactor. Therefore, in the case of the structure shown in FIG. 1, for example, it is possible for the hydrogen consumption quantity measurement system to be additionally provided with a flow meter that measures a flow rate of a gas flowing through the exhaust gas flow path.

It is also possible to employ a hydrogen consumption quantity measurement system that is further provided with a dilution mechanism that dilutes the exhaust gas using a dilution gas so as to create a measurement gas and then supplies the measurement gas to the oxygen concentration sensor, and a reference gas flow path that introduces the dilution gas as a reference gas to the oxygen concentration sensor. A paramagnetic type of sensor, for example, is preferable as this sensor can also be used as the oxygen concentration sensor.

In order to more accurately measure a hydrogen consumption quantity, it is preferable that the dilution mechanism be equipped with a sampling bag that collects the dilution gas, and that the reference gas flow path introduces the reference gas from the sampling bag to the oxygen concentration sensor. If this type of structure is employed, then in cases in which, for example, ambient air is used as the dilution gas, this structure enables the concentration of oxygen contained in the reference gas to be made more stable. This structure is particularly preferable in a case in which a paramagnetic type of sensor is used as the oxygen concentration sensor.

In order to reduce the effects of moisture generated when hydrogen is being oxidized by the oxidation catalyst on the oxygen concentration measurement as much as possible, it is preferable that there be further provided a dehumidifier that is disposed between the oxidation catalyst and the oxygen concentration sensor, and that removes the measurement gas used to dilute the exhaust gas, or moisture contained in the measurement gas which forms a portion of the exhaust gas.

In order to further improve the measurement accuracy, it is preferable that the oxygen concentration sensor be a paramagnetic type of oxygen sensor.

It is also possible to measure the quantity of hydrogen leakage from the hydrogen reactor among the hydrogen contained in the exhaust gas discharged from the hydrogen reactor by measuring a concentration of oxygen in the exhaust gas after this exhaust gas has passed through an oxidation catalyst that performs oxidation using oxygen contained in the exhaust gas, and by measuring the concentration of oxygen in the exhaust gas that has not passed through this oxidation catalyst, and then comparing these oxygen concentrations.

An example of a specific structure that is used to calculate a hydrogen leakage quantity is a structure in which, for example, there are further provided a measurement gas flow path that guides the measurement gas to the oxygen concentration sensor via the oxidation catalyst, a bypass flow path that introduces the exhaust gas to the oxygen concentration sensor without causing it to pass through the oxidation catalyst, and a switching mechanism that switches the flow path connected to the oxygen concentration sensor between the measurement gas flow path and the bypass flow path.

An example of another structure for calculating a hydrogen leakage quantity is a structure in which another oxygen concentration sensor is provided in parallel with the oxygen concentration sensor, and the oxidation catalyst only oxidizes the exhaust gas that has been introduced into either one of the two oxygen concentration sensors.

Moreover, if a structure is employed in which moisture meters are provided upstream and downstream from the oxidation catalyst, then it is possible to determine a quantity of leakage hydrogen from an amount of change between the moisture contained in the exhaust gas on the upstream side and the moisture contained in the exhaust gas on the downstream side of the oxidation catalyst.

If a measurement system is employed that is provided with an oxidation catalyst that is disposed between the hydrogen reactor and the oxygen concentration sensor and that oxidizes hydrogen contained in the exhaust gas using oxygen contained in the exhaust gas, and with a hydrogenation mechanism that adds a predetermined quantity of hydrogen to the measurement gas before the measurement gas flows into the oxidation catalyst, then it is possible to calibrate the oxygen concentration sensor.

[Effects of the Invention]

As has been described above, according to the present invention, it is possible to accurately calculate a total quantity of hydrogen consumed by a hydrogen reactor including leakage hydrogen without having to measure the leakage hydrogen using a separate hydrogen concentration sensor by using an oxygen balance method that does not require any vehicle modifications.

[Brief Description of the Drawings]

[FIG. 1] FIG. 1 is an overall schematic view of a hydrogen consumption quantity measurement system according to an embodiment of the present invention.
[FIG. 2] FIG. 2 is an overall schematic view of a hydrogen consumption quantity measurement system according to a first embodiment of the present invention.
[FIG. 3] FIG. 3 is an overall schematic view of a hydrogen consumption quantity measurement system according to a second embodiment of the present invention.
[FIG. 4] FIG. 4 is an overall schematic view of a hydrogen consumption quantity measurement system according to a third embodiment of the present invention.
[FIG. 5] FIG. 5 is an overall schematic view of a hydrogen consumption quantity measurement system according to a fourth embodiment of the present invention.
[FIG. 6] FIG. 6 shows a graph relating to a calibration of an oxygen concentration sensor according to the fourth embodiment of the present invention.
[FIG. 7] FIG. 7 is an overall schematic view of a hydrogen consumption quantity measurement system according to a fifth embodiment of the present invention.
[FIG. 8] FIG. 8 is an overall schematic view of a hydrogen consumption quantity measurement system according to another embodiment of the present invention.

[Description of the Reference Numerals]

| | |
|---|---|
| 100 | Hydrogen Consumption Quantity Measurement System |
| 100C | Calibration System |
| AIR1 | Air Taken in by Fuel Cell Vehicle |
| AIR2 | Dilution Gas |
| TV | Fuel Cell Vehicle |
| FV | Fuel Cell |
| V | Shut-off Valve |
| 1 | Gas Discharge Path |
| 2 | Sampling Mechanism (Dilution Mechanism) |
| 21 | Measurement Gas Sampling Unit |
| 211 | Dilution Tunnel |
| 212 | Dilution Gas Supply Path |
| 213 | Measurement Gas Sampling Bag |

| 214, 214a, 214b | Measurement Gas Flow Path |
| 216 | Vehicle External Air Flow Path |
| 218 | Bypass Flow Path |
| 22 | Dilution Gas Sampling Unit |
| 221 | Dilution Gas Sampling Bag |
| 222 | Dilution Gas Flow Path |
| 223 | Reference Gas Flow Path |
| 218 | Bypass Flow Path |
| 3 | Oxygen Concentration Sensor |
| 4 | Calculation Unit |
| 5 | Oxidation Catalyst |
| 6 | Dehumidifier |
| 7 | Switching Mechanism |
| 71 | First Switching Valve |
| 72 | Second Switching Valve |
| 73 | Third Switching Valve |
| 74 | Fourth Switching Valve |
| 8 | Hydrogenation Mechanism |
| 81 | Hydrogen Cylinder |
| 82 | Hydrogenation Flow Path |
| 83 | Addition Quantity Control Unit |
| 831 | Flow Rate Control Valve |
| 832 | Valve Controller |
| 833 | Inert Gas Flow Rate Control Valve |
| 84 | Inert Gas Cylinder |
| 85 | Inert Gas Addition Flow Path |
| 9 | Moisture Meter |
| 9a | Pre-dehumidifier Moisture Meter |
| 9b | Post-dehumidifier Moisture Meter |
| 10 | Cylinder |
| 11 | Vibration Alleviation Mechanism |
| 12 | Storage Unit |
| FM | Flow Meter |

[Best Embodiments for Implementing the Invention]

[First Embodiment]

Hereinafter, a hydrogen consumption quantity measurement system according to a first embodiment of the present invention will be described with reference to the drawings. Note that structure that is duplicated in the following embodiments is given the same descriptive symbol in each embodiment and any duplicated description thereof is omitted.

A hydrogen consumption quantity measurement system 100 of the present embodiment determines a hydrogen consumption quantity of a fuel cell FV which is a hydrogen reactor that generates power via an electrochemical reaction of hydrogen, which serves as a fuel gas that is supplied to an anode side, and oxygen which serves as an oxidant gas that is supplied to a cathode side. Note that examples of the fuel cell FV include solid polymer fuel cells, solid oxide fuel cells, and phosphoric acid fuel cells and the like.

[Hydrogen Consumption Quantity Measurement System Basic Structure]

More specifically, the hydrogen consumption quantity measurement system 100 measures the hydrogen consumption quantity of a fuel cell FV that is provided in a fuel cell vehicle TV which is serving as a test body and which is mounted, for example, on a dynamo motor so as to simulate a similar traveling state as if it were traveling on an actual road. The hydrogen consumption quantity measurement system 100 is used, for example, in a fuel consumption evaluation device or the like that calculates the fuel consumption of the fuel cell vehicle TV from the travel distance and the amount of load and the like during travel that are obtained from the dynamo motor, and from the quantity of hydrogen that is consumed which is measured by the hydrogen consumption quantity measurement system 100.

In the present embodiment, the vehicle TV in which the fuel cell FV is provided is used as a test body, however, the test body may instead be a portion of a vehicle in which a fuel cell is provided, or may be the actual fuel cell itself.

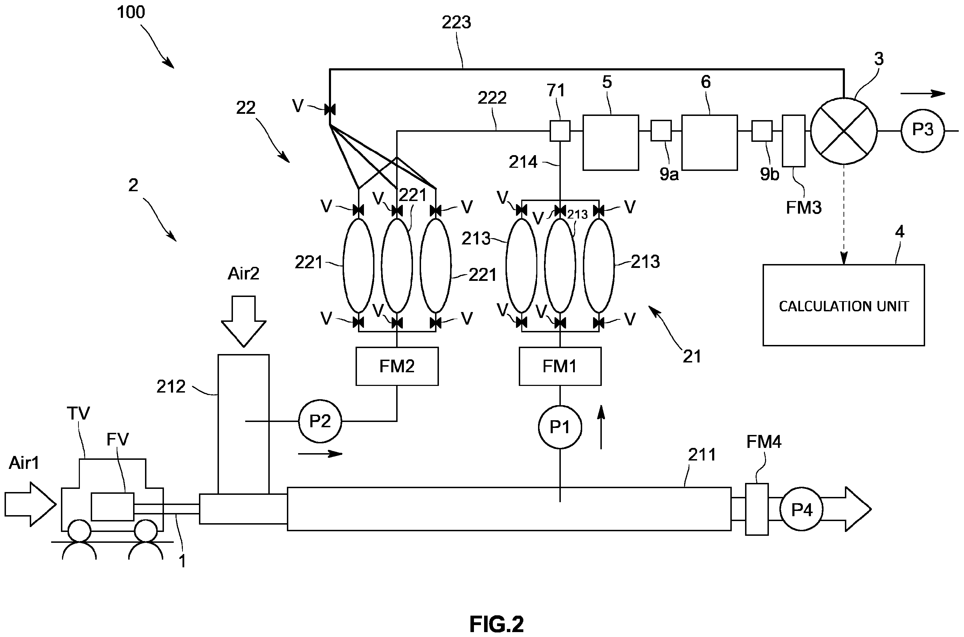

As is shown in FIG. 2, the hydrogen consumption quantity measurement system 100 according to the present embodiment is provided with a gas discharge path 1 through which exhaust gas from the fuel cell FV is discharged, a sampling mechanism 2 that samples the exhaust gas from the gas discharge path 1, an oxygen concentration sensor 3 that measures a concentration of oxygen in the exhaust gas sampled by the sampling mechanism 2, and a calculation unit 4 that calculates a quantity of hydrogen consumption in the fuel cell based on measurement values obtained by the oxygen concentration sensor 3.

Note that Air1 in the drawings denotes air and the like that is taken in by the fuel cell vehicle.

The gas discharge path 1 discharges, for example, exhaust gas from the fuel cell FV to the outside, and may also be provided with a flow meter and a dehumidifier (not shown here).

The sampling mechanism 2 samples the exhaust gas flowing through the gas discharge path 1. In the present embodiment, the sampling mechanism 2 additionally functions as a dilution mechanism to dilute the exhaust gas.

The sampling mechanism 2 of the present embodiment is a constant volume dilution/sampling apparatus (CVS) and is provided with a measurement gas sampling unit 21 that dilutes and samples exhaust gas, and a dilution gas sampling unit 22 that samples only the dilution gas.

The measurement gas sampling unit 21 is provided with, for example, a dilution tunnel 211 that is connected to the gas discharge path 1, a dilution gas supply path 212 that supplies dilution gas in the form of Air2 to the dilution tunnel 211, measurement gas sampling bags 213 that collect measurement gas that has been diluted inside the dilution tunnel 211, a measurement gas flow path 214 that guides the measurement gas collected by the measurement gas sampling bags 213 to the oxygen concentration sensor 3, and a measurement gas flow rate control unit (not shown in the drawings) that controls a flow rate of the measurement gas flowing through the measurement gas flow path 214.

The dilution gas sampling unit 22 is provided with, for example, dilution gas sampling bags 221 that collect the dilution gas captured from the dilution gas supply path 212, a dilution gas flow path 222 that guides dilution gas that has been collected in the dilution gas sampling bags 221 to the oxygen concentration sensor 3, and a dilution gas flow rate control unit (not shown in the drawings) that controls a flow rate of the gas flowing through the dilution gas flow path 222.

The measurement gas flow rate control unit and the dilution gas flow rate control unit are each provided respectively with, for example, a pump P, shut-off valves V, and a flow meter FM and the like, and with an operation control unit or the like (not shown in the drawings) that controls operations of each of these.

In the present embodiment, the measurement gas flow path 214 and the dilution gas flow path 222 merge together on the upstream side of the oxygen concentration sensor 3, and a first switching valve 71 that switches which of these two flow paths is connected to the oxygen concentration sensor 3 is provided at this merging point.

The oxygen concentration sensor 3 measures the respective oxygen concentrations of each one of measurement gas which is the post-dilution exhaust gas that has been sampled by the measurement gas sampling unit 21, dilution gas that has been sampled by the dilution gas sampling unit 22, and, if necessary, reference gas such as ambient air or air supplied from a cylinder or the like.

Note that, in the present specification, the term 'reference gas' refers to gas that is used as a comparison gas in order to make a comparison with the oxygen concentration in the measurement gas that is measured by the oxygen concentration sensor.

This reference gas is used to determine a difference with a reference oxygen concentration, and may be an auxiliary gas that is used in the above-described paramagnetic oxygen sensor, or may be a calibration gas in which the oxygen concentration used for calibration is known and that includes an oxygen sensor other than a paramagnetic oxygen sensor, or may be a comparison gas that is used during a measurement to make a comparison with the air or the like.

In a case in which variations in the oxygen concentration of the air are sufficiently small so as to be within the range of tolerance acceptable for measurement accuracy, then this air can be used for the comparison gas or the auxiliary gas.

In the present embodiment, a paramagnetic oxygen sensor is used as the oxygen concentration sensor 3. Note that a magnetic dumbbell-type oxygen sensor, a galvanic oxygen sensor, or a zirconia oxygen sensor or the like may also be used as the oxygen concentration sensor 3. Note also that it is preferable that the oxygen concentration sensor 3 be calibrated before the measurement process using a calibration gas which is an oxygen gas whose concentration is already known.

The calculation unit 4 functions as a hydrogen consumption quantity calculation unit that calculates a hydrogen consumption quantity in the fuel cell FV using an oxygen balance method based on detection values from the oxygen concentration sensor 3. More specifically, the calculation unit 4 calculates an oxygen consumption quantity in the fuel cell FV from a difference between the oxygen concentrations in the measurement gas and in the dilution gas that are output from the oxygen concentration sensor, and from the flow rate in the dilution tunnel 211, and then calculates the hydrogen consumption quantity from this oxygen consumption quantity.

More specifically, an equation of a reaction between hydrogen and oxygen is shown below (Equation 1). As is shown below in Equation 2, the concentration of the hydrogen that reacts in the fuel cell and is consumed corresponds

to a concentration ($\Delta O_2$ conc.) that is twice a difference between the pre-reaction oxygen concentration (i.e., the oxygen concentration of the dilution gas) and the oxygen concentration in the exhaust gas after the reaction. By then multiplying the dilution exhaust gas quantity (V total) by the hydrogen concentration that has been determined in this manner, it is possible to determine the hydrogen consumption quantity. It is also possible to additionally perform corrections in order to determine more accurate concentration values in the process of performing this equation such as performing a partial pressure correction or the like.

The functions of the calculation unit 4 are able to be performed by a computer that is made up of a CPU, memory, input/output interfaces, and AD converters and the like.

$$2H_2 + O_2 = 2H_2O \qquad\qquad \dots (1)$$

$$\Delta H_{2conc} \ (vol\%) = \Delta O_{2conc} \ (vol\%) \times 2 \qquad\qquad \dots (2)$$

$$\Delta V_{H2} \ (m^3) = \Delta H_{2conc} \ (vol\%) \times V_{total} \ (m^3) \qquad\qquad \dots (3)$$

Note that the volumes shown in Equations (2) and (3) are all volumes in a standard state.

Moreover, $V_{total}$ is a flow rate measured by a flow meter that is mounted immediately in front of a discharge pump that is disposed on the downstream side of the dilution tunnel 211. It is also possible to provide a flow meter that measures the flow rate of the pre-dilution and sampling exhaust gas on the gas discharge path 1, and to determine $V_{total}$ from the flow rate of the exhaust gas flowing through this gas discharge path, and from the flow rate of the dilution gas supplied to the dilution tunnel.

[Characteristic Structure of the Hydrogen Consumption Quantity Measurement System]

It is also possible for the hydrogen consumption quantity measurement system 100 according to the present embodiment to be provided with an oxidation catalyst 5 that is disposed between the fuel cell FV and the oxygen concentration sensor 3, and that oxidizes hydrogen contained in the measurement gas. In the present embodiment, the oxidation catalyst 5 is disposed between the aforementioned first switching valve 71 and the oxygen concentration sensor 3.

The oxidation catalyst 5 oxidizes hydrogen contained in the measurement gas using oxygen contained in this measurement gas, and employs, for example, a platinum (Pt) catalyst or the like. In order to remove water that is generated by the oxidation catalyst 5, it is preferable that a dehumidifier 6 be provided between the oxidation catalyst 5 and the oxygen concentration sensor 3.

It is also possible for a moisture meter 9 that measures the quantity of moisture removed by the dehumidifier 6 to be provided.

If the quantity of moisture removed by the dehumidifier 6 is able to be determined by the moisture meter 9, then it is also possible to determine the amount of change in the moisture partial pressure that occurs as a result of the moisture being removed by the dehumidifier 6. As a result of this, it is possible to more accurately determine the oxygen partial pressure measured by the oxygen concentration sensor 3. In addition, it is also possible to determine the quantity of hydrogen oxidized by the oxidation catalyst 5, in other words, the quantity of leakage hydrogen from this moisture quantity. Note that, in order to perform moisture partial pressure correction on the oxygen concentration measurement values obtained by the oxygen concentration sensor 3, it is only necessary to ascertain the moisture partial pressure of the measurement gas introduced into the oxygen concentration sensor 3. For this reason, it is sufficient if a single moisture meter 9 that is required in order for the moisture partial pressure correction to be performed is provided at least immediately in front of the oxygen concentration sensor 3.

An example of a method that may be employed in order to determine the quantity of moisture removed by the dehumidifier 6 using the moisture meter 9 is a method in which, for example, moisture meters (i.e., a pre-dehumidifier moisture meter 9a and a post-dehumidifier moisture meter 9b) are provided respectively before and after the dehumidifier 6, and the partial pressure of the moisture that has decreased as a result of moisture being removed by the dehumidifier 6 can be determined from the difference in the moisture concentrations measured by these two moisture meters.

In a case in which the dehumidifier is an electronic cooler, then the single moisture meter 9a may be disposed in front of the dehumidifier to serve as the moisture meter 9, and the moisture concentration on the downstream side from this electronic cooler may be calculated from a dew point that corresponds to the cooling set temperature of the electronic cooler.

In other words, in a case in which there is only a small quantity of leakage hydrogen and also only a small

8

quantity of moisture generated by the oxidation catalyst, then it is also possible to omit providing the dehumidifier 6 and to instead provide two dew point meter-type moisture meters.

Note that, as is described above, in a case in which the moisture quantity generated by the oxidation catalyst 5 needs to be measured accurately, it is preferable that a dehumidifier be additionally provided in front of the oxidation catalyst 5.

Furthermore, in the present embodiment, a flow meter FM3 that measures a flow rate of gas flowing into the oxygen concentration sensor 3 is provided between the dehumidifier 6 and the oxygen concentration sensor 3.

The dilution gas sampling unit 22 is additionally provided with a reference gas flow path 223 that guides dilution gas collected by the dilution gas sampling bags 221 to the oxygen concentration sensor 3 as the above-described reference gas. The reference gas flow path 223 is used when an oxygen concentration sensor that requires auxiliary gas and reference gas such as a paramagnetic oxygen sensor or the like is used, and is not used for other types of sensors.

The calculation unit 4 acquires information relating to the load of the fuel cell, the oxygen concentration measured by the oxygen concentration sensor 3, and the flow rate of the gas flowing into the dilution tunnel 211 that was measured by the flow meter FM4, and then determines from the oxygen concentration the weight of the oxygen consumed by the fuel cell FV The calculation unit 4 then calculates the hydrogen consumption quantity based on this oxygen weight.

In addition, the calculation unit 4 acquires the moisture quantities from the above-described moisture meters 9a and 9b, and then determines the amount of change between the moisture partial pressure in front of the dehumidifier 6 and the moisture partial pressure after the dehumidifier 6. The calculation unit 4 then calculates the quantity of moisture removed by the dehumidifier.

[Effects Obtained from the First Embodiment]

According to the hydrogen consumption quantity measurement system 100 of the present embodiment having the above-described structure, because leakage hydrogen discharged from the fuel cell FV consumes oxygen contained in the measurement gas and is oxidized by the oxidation catalyst 5, the hydrogen leakage quantity can be reflected in the oxygen concentration that is measured by the oxygen concentration sensor 3. As a result, it is possible to measure the total hydrogen consumption quantity in the fuel cell FV without having to measure the leakage hydrogen using a separately provided hydrogen concentration sensor or the like.

Moreover, because the dehumidifier 6 is provided between the oxidation catalyst 5 and the oxygen concentration sensor 3, in a case in which a large quantity of moisture is generated on the downstream side of the oxidation catalyst, it is possible to prevent condensation from forming in the gas flow path, and to also reduce the effects on the measurement by the oxygen concentration sensor 3 that are caused by such moisture.

Moreover, because the moisture meter 9a is provided on the downstream side of the oxidation catalyst 5, in a case in which another moisture meter (not shown in the drawings) is additionally provided on the upstream side of the oxidation catalyst 5, then it is possible for the leakage hydrogen quantity to also be calculated by the calculation unit 4 from the amount of change between the moisture quantity upstream from the oxidation catalyst 5 and the moisture quantity downstream from the oxidation catalyst 5 as measured by the moisture meter 9a.

Because dilution gas collected in the dilution gas sampling bags 221 that are provided in a constant volume dilution/sampling apparatus that is serving as the sampling mechanism 2 is used as the auxiliary gas for the paramagnetic oxygen sensor, it is possible for the measurement accuracy in the oxygen concentration sensor 3 to be improved over the conventional technology. The reasons for this are thought to be (1) the fact that it is possible to reduce to as great a degree as possible the effects on the measurement values from variations in the oxygen concentration in air compared to a case in which unmodified ambient air is used as the auxiliary gas, and (2) the fact that, by utilizing the dilution gas used to dilute the measurement gas as the auxiliary gas, it is possible to suppress any drift in the measurement values that is caused by differences in the base gas.

Furthermore, because a paramagnetic type of oxygen concentration sensor is used as the oxygen concentration sensor 3, it is possible for the total hydrogen consumption quantity in the fuel cell FV to be measured even more accurately. Because leakage hydrogen contained in exhaust gas is oxidized by an oxidation catalyst and removed, it is possible to also suppress the effects on an oxygen concentration measurement from the magnetic susceptibility of the hydrogen, and to thereby further improve the measurement accuracy when an oxygen concentration is measured using a paramagnetic type of oxygen concentration sensor.

[Second Embodiment]

Next, a hydrogen consumption quantity measurement system of a second embodiment will be described.

A hydrogen consumption quantity measurement system 100A of the second embodiment also functions as a hydrogen leakage quantity measurement system that calculates the above-described hydrogen leakage quantity in addition to the total hydrogen consumption quantity.

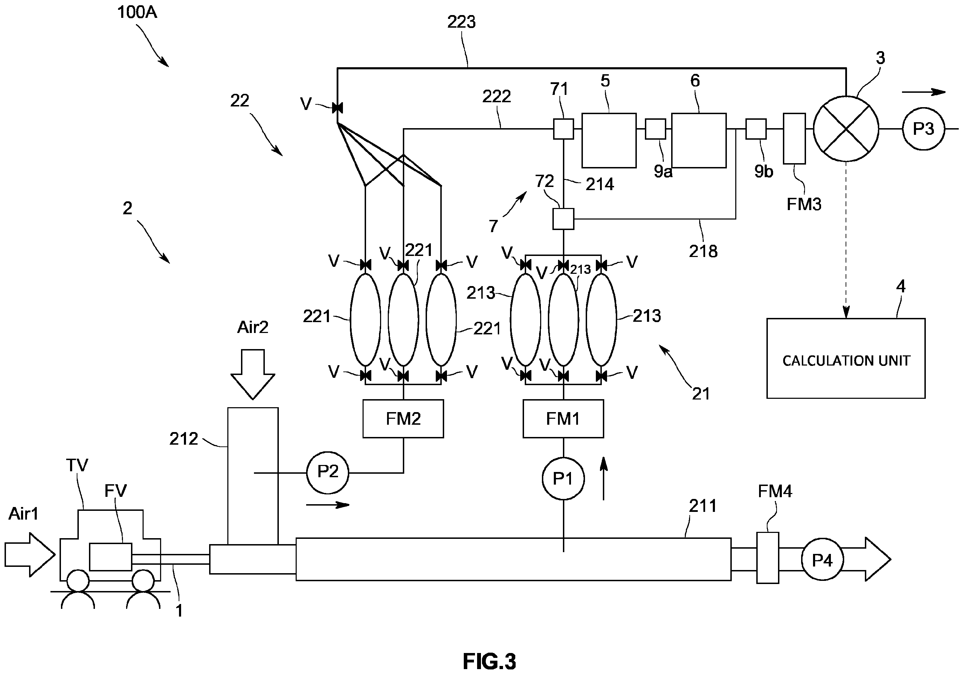

More specifically, as is shown in FIG. 3, in addition to the structure of the above-described embodiment, the hydrogen consumption quantity measurement system 100A is further provided with a bypass flow path 218 that guides the measurement gas to the oxygen concentration sensor 3 without causing it to pass through the oxidation catalyst 5, and with a switching mechanism 7 that switches the flow path that is connected to the oxygen concentration sensor 3 between the measurement gas flow path 214 and the bypass flow path 218.

The switching mechanism 7 is provided, for example, with a second switching valve 72 that is formed by a 3-way valve or the like, and with a switching control unit (not shown in the drawings) that controls operations of the second switching valve 72. The functions of this switching control unit are able to be performed by a computer that is made up of a CPU, memory, input/output interfaces, and AD converters and the like, and this computer may also be used to control the above-described first switching valve.

A desired quantity of the measurement gas that has been sampled, for example, by the same measurement gas sampling bag 213 is supplied by the switching mechanism 7 to the measurement gas flow path 214, and an oxygen concentration that takes into account the hydrogen leakage quantity is measured. If the remaining measurement gas is then diverted to the bypass flow path 218, it is possible to measure an oxygen concentration that does not take into account the hydrogen leakage quantity.

The calculation unit 4 calculates the hydrogen leakage quantity based on the oxygen concentration in the measurement gas that has passed through the oxidation catalyst 5 and has been measured in the manner described above, and the oxygen concentration in the measurement gas that has not passed through the oxidation catalyst 5. In this case, the calculation unit 4 may be said to function as a hydrogen leakage quantity calculation unit.

[Effects Obtained from the Second Embodiment]

According to the hydrogen consumption quantity measurement system 100A of the present embodiment that is formed in the above-described manner, by switching the flow path that is connected to the oxygen concentration sensor 3 between the measurement gas flow path 214 and the bypass flow path 218, it is possible to measure the difference between the oxygen concentration of the measurement gas that has passed through the oxidation catalyst 5 and the oxygen concentration of the measurement gas that has not passed through the oxidation catalyst 5. From this difference between the oxygen concentrations it is possible to calculate the quantity of leakage hydrogen from the above-described Equation (1), Equation (2), and Equation (3).

[Third Embodiment]

Next, a hydrogen consumption quantity measurement system of a third embodiment will be described.

A hydrogen consumption quantity measurement system 100B of the third embodiment also functions as a hydrogen leakage quantity measurement system that, in the same way as the second embodiment, calculates the above-described hydrogen leakage quantity in addition to the total hydrogen consumption quantity.

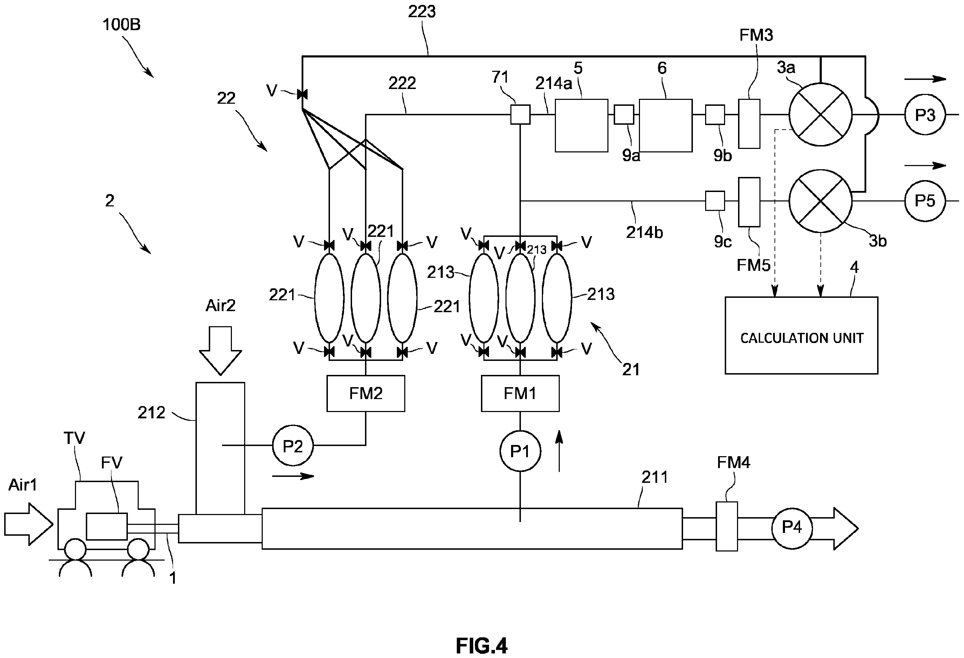

More specifically, as is shown in FIG. 4, in addition to the structure of the above-described first embodiment, the hydrogen consumption quantity measurement system 100B is further provided with an additional oxygen concentration sensor 3. Moreover, the hydrogen consumption quantity measurement system 100B is formed in such a way that the measurement gas flow path 214 is split into two flow paths on the upstream side of the oxidation catalyst 5 so that a measurement gas flow path 214a that passes through the oxidation catalyst 5 is connected to one oxygen concentration sensor 3a, and a measurement gas flow path (that does not include a catalyst or a dehumidifier) 214b that does not pass through the oxidation catalyst 5 is connected to another oxygen concentration sensor 3b. The calculation unit 4 calculates the hydrogen leakage quantity based on the oxygen concentration in the measurement gas that has passed through the oxidation catalyst 5 and has been measured in the manner described above, and the oxygen concentration in the measurement gas that has not passed through the oxidation catalyst 5. In this case, the calculation unit 4 may be said to function as a hydrogen leakage quantity calculation unit. In a case in which a significant difference between the sensitivities of the two oxygen sensors is evident, using a sensitivity ratio $\alpha$ of the sensitivity of the other oxygen concentration sensor 3b relative to the sensitivity of the one oxygen concentration sensor 3a, it is also possible to determine the hydrogen leakage quantity using the above-described Formula (3) together with Formula (4) shown below.

$$\Delta H_{2conc.} \text{ (leakage concentration vol\%)} = 2\Delta O_{2conc.} \text{ (concentration value from 3a vol\%)} \times$$

$$\alpha - 2\Delta O_{2conc.} \text{ (concentration value from 3b vol\%)} \qquad \ldots (4)$$

$$\Delta V_{H2} \text{ (m}^3\text{)} = \Delta H_{2conc.} \text{ (vol\%)} \times V_{total} \text{ (m}^3\text{)} \qquad \ldots (3)$$

Wherein, in a case in which no significant difference between the sensitivities of the two oxygen sensors is evident, then $\alpha$ can be regarded as 1.

[Effects Obtained from the Third Embodiment]

According to the hydrogen consumption quantity measurement system 100B of the present embodiment that is formed in the above-described manner, in addition to the effects obtained from the second embodiment, it is also possible to simultaneously measure both the measurement gas that has passed through the oxidation catalyst 5 and the measurement gas that has not passed through the oxidation catalyst 5.

Moreover, in the present embodiment, because the moisture meters 9b and 9c are provided respectively immediately before the two oxygen concentration sensors 3a and 3b, it is possible to perform moisture partial pressure correction respectively on each of the two oxygen concentration sensors 3a and 3b by using the moisture partial pressures measured by these two moisture meters 9b and 9c. Because of this, it becomes possible to determine a hydrogen leakage quantity even more accurately.

[Fourth Embodiment]

Next, a hydrogen consumption quantity measurement system 100C (i.e., a calibration system 100C) of a fourth embodiment will be described.

A calibration system 100C of the fourth embodiment calibrates the oxygen concentration sensor 3 that is also used in each of the above-described embodiments as well.

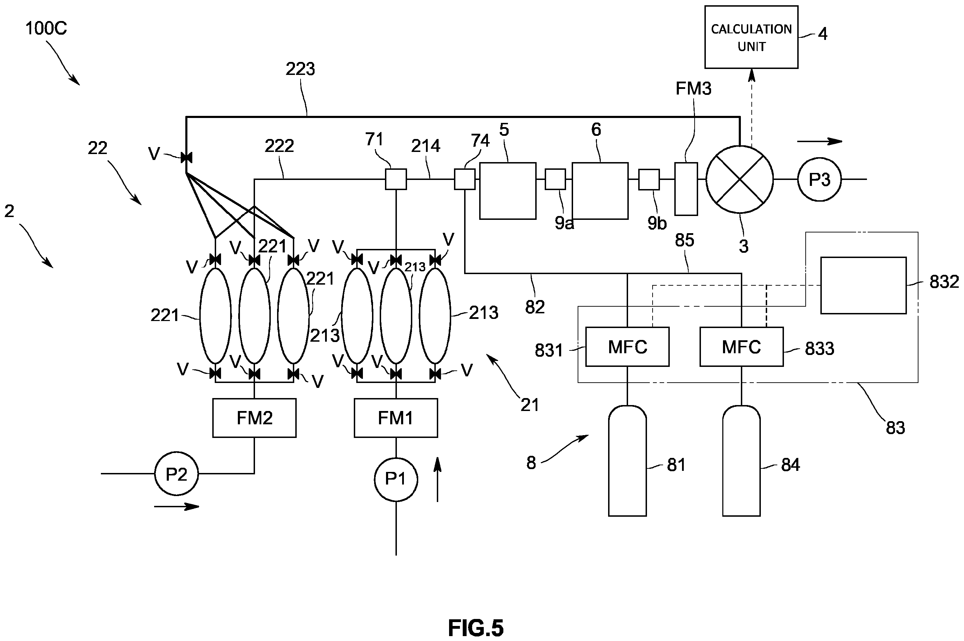

More specifically, as is shown in FIG. 5, the calibration system 100C is additionally provided with a hydrogenation mechanism 8 that adds a predetermined quantity of hydrogen to the measurement gas flowing through the above-described measurement gas flow path 214.

The hydrogenation mechanism 8 is provided with a hydrogen cylinder 81 that stores hydrogen whose concentration is already known, a hydrogenation flow path 82 that is connected to the hydrogen cylinder 81 and the measurement gas flow path 214 and that adds hydrogen supplied from the hydrogen cylinder 81 to the measurement flow path 214 on the upstream side of the oxidation catalyst 5, and an addition quantity control unit 83 that controls a flow rate of the hydrogen flowing through the hydrogenation flow path 82. It is also possible for a fourth switching valve 74 to be provided at a merge point between the measurement gas flow path 214 and the hydrogenation flow path 82. The addition quantity control unit 83 is provided, for example, with a flow rate control valve 831 and a valve controller 832 that controls a valve opening of this flow rate control valve 831. The functions of this valve controller 832 are able to be performed by a computer that is made up of a CPU, memory, input/output interfaces, and AD converters and the like. If the supply rate of the hydrogen being supplied from the hydrogen cylinder 81 is changed while the flow rate of the measurement gas being supplied to the oxygen concentration sensor 3 is held constant, then it is possible to change the concentration of hydrogen contained in the gas measured by the oxygen concentration sensor 3 in successive steps.

In a case in which only an extremely small amount of hydrogen is to be added by the hydrogenation mechanism 8, then it is also possible for the hydrogen to be added after it has been diluted using an inert gas that does not have any effect on the oxygen concentration measurement (for example, nitrogen gas or the like). In this case, as is shown in FIG. 5, it is also possible for there to be additionally provided a dilution inert gas cylinder 84 that contains an inert gas used for diluting, an inert gas addition flow path 85 that connects the dilution inert gas cylinder 84 to the hydrogenation flow path 82, and an inert gas flow rate control valve 833 that is provided on the inert gas addition flow path 85 and controls a flow rate of the inert gas flowing through the inert gas addition flow path 85. The inert gas flow rate control valve 833 may be controlled by the above-described valve control unit 832, or an independent inert gas valve control unit may instead be provided.

[Effects Obtained from the Fourth Embodiment]

The calibration system 100C of the present embodiment that is formed in the above-described manner measures the oxygen concentration via the oxygen concentration sensor 3 while simultaneously increasing or decreasing in steps the quantity of hydrogen being added to the measurement gas flow path 214 using the addition quantity control unit 83. If this type of procedure is employed, then by measuring the difference between the oxygen concentration of the calibration air whose oxygen is consumed by the oxidation catalyst 5 so as to intentionally decrease the oxygen concentration, and the oxygen concentration of the air serving as a reference gas, while simultaneously changing in steps the quantity of hydrogen that is added, it is possible to correct the rectilinearity of the measurement results from the oxygen concentration sensor 3 in the vicinity of the concentration of oxygen in air.

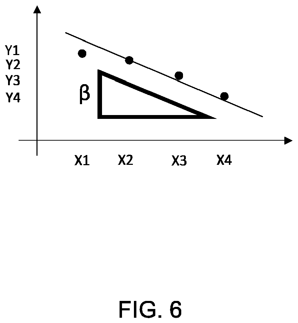

More specifically, for example, the above-described calculation unit 4 predicts the amount of change in the oxygen concentration that ought to be measured by the oxygen concentration sensor 3 based on the quantity of hydrogen that was added by the hydrogenation mechanism 8, and then creates a calibration curve using this concentration and using the amount of change in the oxygen concentration that was actually measured by the oxygen concentration sensor 3, and consequently obtains an inclination β from this calibration curve. The measurement results obtained by the oxygen concentration sensor 3 can then be corrected using the inclination β as a sensitivity coefficient or the like of the oxygen concentration sensor 3. As a result, it is possible to improve even further the accuracy of a measurement of the quantity of hydrogen consumed by a fuel cell made using the oxygen concentration sensor 3. A specific example of the calibration method according to the present embodiment is a method in which, for example, by changing the quantity of added hydrogen in steps such as those shown in Table 1, it is possible to obtain a calibration curve such as that shown in FIG. 6. It is desirable that the number of steps in which the quantity of added hydrogen is changed in order to obtain this calibration curve be not less than two.

The method of calculating the amount of change in the oxygen concentration that is predicted from the quantity of added hydrogen takes into account a flow rate L1 in the measurement gas flow path 214, a flow rate L2 in the hydrogenation flow path 82, a flow rate L3 in the inert gas addition flow path 85, and a hydrogen purity γ [vol%] recorded in the hydrogen cylinder, and is shown by the following Equation (5).

$$\Delta C_{O2} \text{ (an amount of change [g/cm}^3\text{] in the oxygen concentration predicted from the}$$

$$\text{hydrogen addition quantity)} = 1/2 \times (\gamma/100) \times (L2/(L1 + L2 + L3)) \quad \ldots (5)$$

[Table 1]

| Step number | 1 | 2 | 3 | 4 |
|---|---|---|---|---|
| Amount of change in the oxygen concentration predicted from the hydrogen addition quantity | X1 | X2 | X3 | X4 |
| Amount of change in the oxygen concentration actually measured by the oxygen sensor 3 | Y1 | Y2 | Y3 | Y4 |

[Fifth Embodiment]

Next, a hydrogen consumption quantity measurement system of a fifth embodiment will be described.

A hydrogen consumption quantity measurement system 100D of the fifth embodiment does not use an exhaust gas dilution mechanism, and also functions as a hydrogen leakage quantity measurement system that calculates the above-described hydrogen leakage quantity in addition to the total hydrogen consumption quantity.

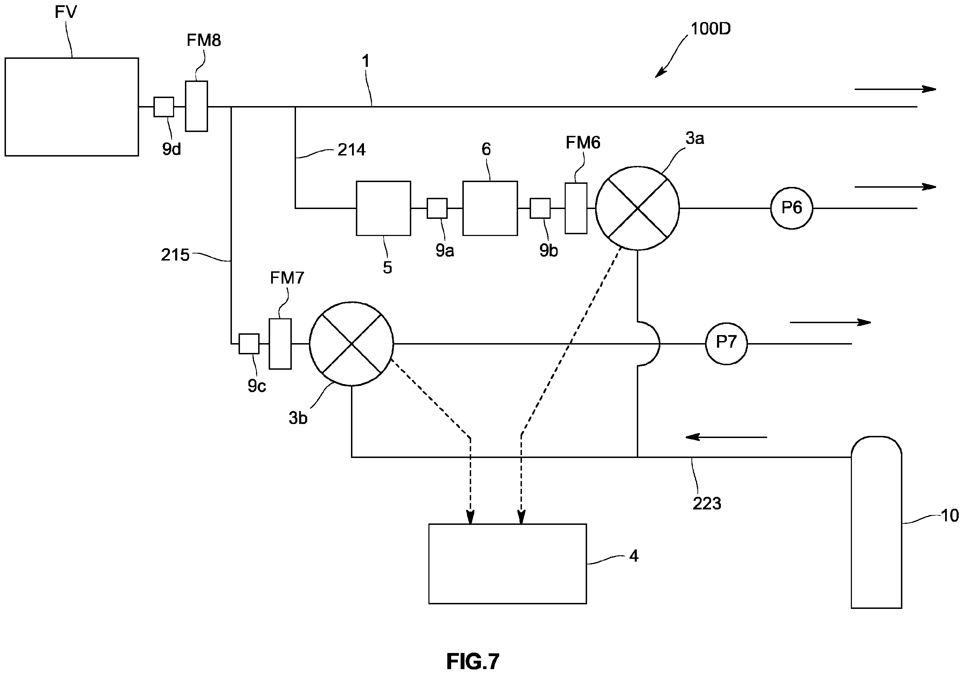

As is shown in FIG. 7, in a case in which a dilution mechanism is not used, then it is also possible for the measurement gas flowing through the measurement gas flow path 214 that branches off from the gas discharge path 1 to be measured directly by the oxygen concentration sensor 3.

In the case shown in FIG. 7, there are provided two oxygen concentration sensors 3, and two measurement gas flow paths 214 and 215 that respectively connect these oxygen concentration sensors 3a and 3b to the gas discharge path 1. Of these two measurement gas flow paths 214 and 215, the oxidation catalyst 5, moisture meter 9a, dehumidifier 6, and an additional moisture meter 9b are provided on the one measurement gas flow path 214. On the other measurement gas flow path 215 are provided the moisture meter 9c and the flow meter FM.

Moreover, it is also possible for air to be used as the reference gas in these two oxygen concentration sensors 3a and 3b and, as is shown in FIG. 7, for a cylinder 10 that is filled with air to be used. In the case shown in FIG. 7, it is preferable that reference gas flow paths are connected from the single cylinder 10 to the two oxygen concentration sensors 3a and 3b.

According to the hydrogen consumption quantity measurement system shown in FIG. 7, it is possible to calculate a hydrogen consumption quantity, for example, in the following manner.

Firstly, a case in which the oxygen concentration of the reference gas contained in the cylinder 10 shown in FIG. 7 is known will be described. In this case, because a reference gas having a known oxygen concentration is being used, the oxygen concentration values (i.e., instantaneous values) shown by the respective oxygen concentration sensors are values having the same level of reliability as the oxygen concentration of the reference gas.

A relationship between an oxygen concentration $O_2$ (conc3b) obtained by the oxygen concentration sensor 3b, an oxygen concentration $O_2$ (conc.Air) of the ambient air, and an amount of change $\Delta O_2$ (FV) in the oxygen concentration due to oxygen being consumed by the chemical reaction generated as a result of the test body FV generating power can be expressed as in the following Equation (6).

$$O_2 \text{ (conc3b)} = O_2 \text{ (conc.Air)} - \Delta O_2 \text{ (FV)} \qquad \dots (6)$$

Moreover, a relationship between an oxygen concentration $O_2$ (conc3a) obtained by the oxygen concentration sensor 3a, the oxygen concentration $O_2$ (conc.Air) of the ambient air, the amount of change $\Delta O_2$ (FV) in the oxygen concentration, and a quantity of oxygen $\Delta O_2$ (leak) that is consumed by reacting with leakage hydrogen in the test body FV can be expressed as in the following Equation (7).

$$O_2 \text{ (conc3a)} = O_2 \text{ (conc.Air)} - \Delta O_2 \text{ (FV)} - \Delta O_2 \text{ (leak)} \qquad \dots (7)$$

If Equation (7) is subtracted from Equation (6), then $\Delta O_2$ (leak) can be calculated in the manner shown by the following Equation (8).

$$O_2 \text{ (conc3b)} = O_2 \text{ (conc3a)} = \Delta O_2 \text{ (leak)} \qquad \dots (8)$$

Furthermore, in a case in which a correction coefficient $\alpha$ that corrects the sensitivities of the respective oxygen concentration sensors 3a and 3b is used, then Equation (8) is modified in the following manner.

$$O_2 \text{ (conc3b)} \times \alpha - O_2 \text{ (conc3a)} = \Delta O_2 \text{ (leak)} \qquad \dots (9)$$

Next, a case in which the oxygen concentration of the reference gas contained within the cylinder 10 shown in FIG. 7 is unknown will be described.
In this case, the oxygen concentration value that is measured by the above-described oxygen concentration sensors 3a and 3b and then displayed (i.e., the display value) forms relative concentration information that is compared with the reference gas whose oxygen concentration is unknown (here, the oxygen concentration of this reference gas is taken as X). However, if the procedure described below, for example, is followed, then it is possible to calculate the hydrogen consumption quantity and the leakage hydrogen quantity in the test body FV based on the display values from the oxygen concentration sensors 3a and 3b.
The display values (Display) of the oxygen concentrations in the respective oxygen concentration sensors can be expressed as in the following Equation (10) and Equation (11).

$$(\text{Display 3a}) = X - (O_2(\text{conc.Air}) - \Delta O_2(\text{FV}) - \Delta O_2(\text{leak})) \qquad \dots (10)$$

$$(\text{Display 3b}) = X - (O_2(\text{conc.Air}) - \Delta O_2(\text{FV})) \qquad \dots (11)$$

For this reason, as is calculated in Equation (8) and Equation (9) given above, by determining the difference between the display values of the oxygen concentration sensors 3a and 3b, it is possible to cancel out any unknown concentration items in the reference gas, and to determine the oxygen quantity $\Delta O_2(\text{leak})$ that has reacted with the leakage hydrogen in the test body FV and been consumed.
More specifically, the difference between the display values of the oxygen concentration sensors 3a and 3b is determined. In other words, by subtracting Equation (10) from Equation (11), Equation (12) is obtained.

$$(\text{Display 3b}) - (\text{Display 3a}) = \Delta O_2(\text{leak}) \qquad \dots (12)$$

As is shown in Equation (12), even in a case in which absolute values of the oxygen concentrations measured by the respective oxygen concentration sensors 3a and 3b are unknown, it is still possible to determine the oxygen quantity $\Delta O_2(\text{leak})$ that has reacted with the leakage hydrogen in the test body FV and been consumed.
The above-described Equation (9) and Equation (12) determine an instantaneous value of each oxygen concentration that corresponds to the hydrogen leakage quantity in the test body FV
Note that, in the case of the structure shown in FIG. 7, in a case in which the change over time in the concentration of oxygen in the air during the testing of the test body FV is not regarded as a small enough change that it can be ignored, then it is also possible to measure in advance the concentration of oxygen in the air ($O_2(\text{conc.Air})$). For example, it is possible to use the oxygen concentration sensor 3b to measure air that has traveled in reverse so as to be taken in through the exhaust port of the exhaust pipe 1 when the test body FV is not in operation.

In a case in which an operator wishes to also measure at the same time the concentration of oxygen in the air during the testing of the test body FV, then it is also possible to prepare a third oxygen concentration sensor 3c (not shown in the drawings).

In a case in which the third oxygen concentration sensor 3c (not shown in the drawings) is provided and atmospheric gas or the like whose oxygen concentration is unknown is used as the reference gas, then the display value of the oxygen concentration measured by this oxygen concentration sensor 3c can be shown by the following Equation (13).

$$(\text{Display 3c}) = X - O_2(\text{conc.Air}) \qquad \dots (13)$$

By subtracting Equation (11) from Equation (13), in the same way as in Equation (12), it becomes possible to eliminate the concentration X and $O_2(\text{conc.Air})$ items. As a result, it is possible to determine the concentration of oxygen that has chemically reacted in the test body FV and been consumed using the following Equation (14).

$$(\text{Display 3c}) - (\text{Display 3b}) = \Delta O_2(\text{FV}) \qquad \dots (14)$$

Hitherto, a method of calculating an instantaneous value for an oxygen concentration has been described, however, it is also possible for the total hydrogen consumption quantity in a case in which the structure shown in FIG. 7 is used to be determined in the following manner.

Instantaneous concentration values of $\Delta O_2(\text{FV})$ and $\Delta O_2(\text{leak})$ at a particular time t are written respectively as $\Delta O_2(\text{FV})\_t$ [vol%] and $\Delta O_2(\text{leak})\_t$ [vol%]. If the instantaneous exhaust quantity of the test body FV is taken as $V\_t[m^3]$ (i.e., the instantaneous value of a flow meter FM8), then the total hydrogen consumption quantity during a testing time T of 0~t can be expressed by the following Equation (15).

$$O_2\,\text{total}\ (\text{m}^3)\ =\ \int_0^t (V_T \times (\Delta O_2(FV)_T + \Delta O_2(leak)_{\_T})dT \qquad (15)$$

By applying a total oxygen quantity $O_2$total (m$^3$) that has reacted in the test body FV including the oxygen that has reacted with the leakage hydrogen to Equation (2), it is possible to determine the total quantity of hydrogen consumption including the leakage hydrogen when the testing time T was 0~t.

It goes without saying that, by performing an integration in the same way, it is also possible to calculate just the leakage hydrogen quantity.

Note that, in the above-described hydrogen consumption quantity calculation method, the moisture meter 9b and the flow meter FM6 provided in the dehumidifier 6 and the oxygen concentration sensor 3a shown in FIG. 7 are not essential parts of the structure, and it is also possible for these parts to be omitted.

Effects Obtained from the Fifth Embodiment]

According to the hydrogen consumption quantity measurement system 100D of the present embodiment that is formed in the above-described manner, because leakage hydrogen discharged from the fuel cell FV by the oxidation catalyst 5 consumes oxygen contained in the measurement gas and is oxidized, it is possible for the hydrogen leakage quantity to be reflected in the instantaneous value of the oxygen concentration measured by the oxygen concentration sensor 3. As a result, it is possible to measure an instantaneous value of the hydrogen consumption quantity in the fuel cell FV without having to measure the leakage hydrogen using a separately provided hydrogen concentration sensor or the like.

Moreover, according to the hydrogen consumption quantity measurement system 100D of the present embodiment, because a dilution structure is not employed, it is possible to measure an instantaneous value of the difference between the concentration of oxygen in the measurement gas that has passed through the oxidation catalyst 5 and the concentration of oxygen in the measurement gas that has not passed through the oxidation catalyst 5.

[Additional Embodiments]

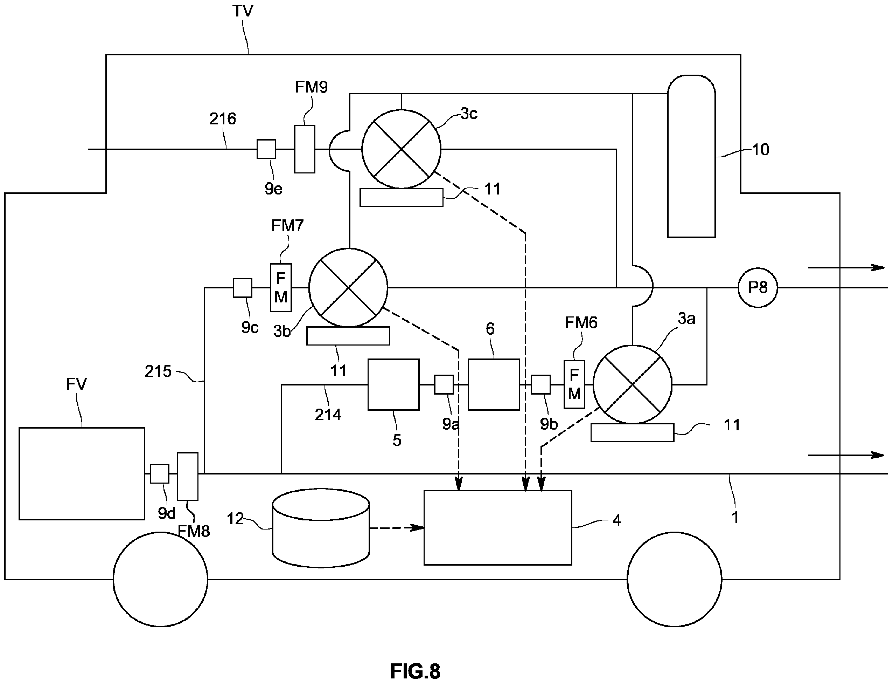

The hydrogen consumption quantity measurement system according to the present invention may also be a vehicle on-board type of system that measures the hydrogen consumption quantity of a fuel cell vehicle when that vehicle is traveling on an actual road. In this case, as is shown in FIG. 8, it is possible, for example, for three oxygen concentration sensors to be provided. In the same way as in the fifth embodiment, a structure may be employed in which two of these three oxygen concentration sensors are connected to the gas discharge path 1 and directly measure an instantaneous value of the oxygen concentration of the exhaust gas sampled from the vehicle exhaust pipe, and an instantaneous value of the oxygen concentration that includes the leakage hydrogen concentration. Furthermore, the remaining oxygen concentration sensor may be connected to a vehicle external air flow path 216 through which flows ambient air (Air1) from outside the vehicle, and may measure the oxygen concentration of the vehicle external air at the same time as the measurements are being made by the aforementioned two oxygen concentration sensors. An instantaneous value of the hydrogen consumption quantity and a hydrogen leakage quantity may then be calculated from these three types of oxygen concentrations.

Moreover, in a case in which this type of vehicle on-board system is employed, in a case in which the measurement values of the oxygen concentration sensors are affected by vibrations from the vehicle, it is possible for vibration damping mechanisms 11 such as shock-absorbing pads or the like that dampen vibration from the vehicle to be additionally provided. It is also possible for a storage unit that stores the temperature, humidity, and air pressure outside the vehicle, as well as the vehicle speed and travel distance information to be additionally provided.

Using the hydrogen consumption quantity obtained by means of the present invention and the distance traveled by a vehicle, it becomes possible to calculate a fuel efficiency. The fuel efficiency can be calculated using, for example, the following equation.

$$\text{(distance traveled by vehicle [km])} \ / \ \text{(quantity of hydrogen consumed [kg])} = \text{fuel efficiency [km/kg]}$$

Note that, in a case in which a magnetic oxygen concentration sensor is used as the oxygen concentration sensor of a vehicle on-board hydrogen consumption quantity measurement system such as that shown in FIG. 8, as is described above, it is not absolutely essential that three oxygen concentration sensors be provided. For example, if ambient air is used as the auxiliary gas of the magnetic oxygen concentration sensor, then it is possible to determine the hydrogen consumption quantity of a test body using only a single oxygen concentration sensor.

If a structure in which two magnetic oxygen concentration sensors are provided is employed, then this structure is preferable as it enables the hydrogen leakage quantity to also be determined.

In the above-described embodiments, a hydrogen consumption quantity and hydrogen leakage quantity are calculated by a calculation unit provided in the hydrogen consumption quantity measurement system, however, the present invention is not limited to this and it is also possible for a user to calculate the hydrogen consumption quantity and hydrogen leakage quantity and the like based on measurement values obtained from the oxygen concentration sensors and the like.

If concentration values containing few temporal variations are obtained using, for example, a method in which concentration signals that have been stabilized via noise processing from among a plurality of concentration signal data acquired at fixed time intervals are obtained, or in which moving average values of the concentration signals are obtained, then it is possible to further improve the accuracy when measuring an oxygen concentration. An example of this noise processing is a method in which in a process in which an average value of the acquired plurality of concentration signal data is calculated and this average value is obtained as the concentration value. Next, concentrations signals that are separated by a predetermined ratio or more from this average value are excluded from the calculation, and the average value of the remaining concentration signal data is taken as the concentration value. In particular, in cases such as those in the above-described first through fourth embodiments, because dilution gas collected in a dilution gas sampling bag provided in a constant volume dilution/sampling apparatus is used, by combining these methods with the above-described noise processing it is possible to sufficiently stabilize a concentration signal output from the oxygen concentration sensors. On the other hand, for example, because a main purpose of the method according to the fifth embodiment is to obtain concentration values having superior responsiveness in real time, it is also possible to perform the above-described noise processing and smoothing processing to smooth concentration signal data obtained from moving averages, however, it is not essential that these processings be performed.

The calibration of the oxygen concentration sensor may be achieved by performing the calibration task after narrowing down the assumed oxygen concentration range, or by performing the calibration task in a concentration range extending from a completely oxygen-free state to an oxygen concentration equivalent to that of air. More specifically, as in the first through fourth embodiments, in a case in which a constant volume dilution/sampling apparatus is provided and the oxygen concentration range that is measured by the oxygen concentration sensor is narrowed down somewhat, then it is also possible to perform the calibration task by setting the oxygen concentration range to between 18-21% or the like. Moreover, as in the fifth embodiment, in a case in which exhaust gas from the fuel cell is supplied directly, because it is anticipated that there will be large variations in the oxygen concentration, it is possible to perform the calibration task by setting the oxygen concentration to between 0-21%. Note that because the resolution of the concentration values displayed by the oxygen concentration sensor increases as the concentration range is narrowed down, it is preferable that the oxygen concentration range where the calibration task is performed be made as narrow as possible.

It should also be noted that, in the methods according to the above-described first through fourth embodiments, the sample gas is diluted, however, the method according to the fifth embodiment is a method in which the sample gas is not diluted. Because of structural variations of this type, in each of these methods the amount of change in the oxygen concentrations and the measurement range thereof differ in accordance with the magnitude of the dilution ratio. Because of this, depending on which method is used, it may be necessary to suitably adjust the display values of the measurement range and oxygen concentrations of the oxygen concentration sensor. Therefore, if, for example, an oxygen concentration sensor having a function that enables the measurement range to be adjusted automatically is used, then it becomes possible for a single oxygen concentration sensor to be used to create a hydrogen consumption quantity measurement system capable of being used in both the methods according to the first through fourth embodiments and the method according to the fifth embodiment.

Note that the fact that it is possible to use air or a dilution gas as the reference gas in the oxygen balance method without using a standard gas, which has conventionally been considered indispensable, is also one of the principal effects of the present invention, and an oxidation catalyst is not absolutely essential structure in order for this effect to be demonstrated.

In each of the above-described embodiments, a case is described in which the test body is an automobile provided with a fuel cell, however, the present invention is not limited to this and it is also possible, for example, for the test body to be another type of vehicle such as a train or the like or a ship that is provided with a fuel cell, or to be a portion of these, or to be just the actual fuel cell itself.

In other words, the test body is not limited to being an object provided with a fuel cell, and may be a hydrogen engine vehicle in which a hydrogen engine is provided as a hydrogen reactor, or a portion thereof, or may be the actual hydrogen engine itself.

Because a hydrogen engine employs, as motive power, energy obtained from the combustion reaction when hydrogen being used as fuel combusts with oxygen in the air in the same way as a fuel cell, a hydrogen engine can be used as a subject in which the hydrogen consumption quantity can be determined using the oxygen balance method. Moreover, in a hydrogen engine as well, not all of the hydrogen input for combustion reacts with oxygen in the engine, and a portion thereof remains within the engine even after the combustion process as unreacted hydrogen, and this unreacted hydrogen forms exhaust gas that is discharged from the engine. This is because the same problems as the problems in a fuel cell, namely, problems such as errors occurring in the total hydrogen consumption quantity measured using the oxygen balance method, are generated due to this unreacted hydrogen.

In order to further improve the accuracy of a hydrogen consumption quantity measurement made using a hydrogen reactor, it is preferable that a reaction in the oxidation catalyst between hydrocarbon and oxygen in the air be considered. For example, the total hydrocarbon (THC) concentration in the air is measured, and the concentration of oxygen that is consumed by the reaction with the hydrocarbon is calculated. By using the result of this calculation when calculating the hydrogen consumption quantity, it is possible to more accurately determine the hydrogen consumption quantity in the hydrogen reactor.

Moreover, in a hydrogen engine, because there are cases in which oxygen consumption occurs due to thermal NOx being generated, it is possible to more accurately calculate the hydrogen consumption quantity in a hydrogen engine by measuring NOx in the exhaust gas and then correcting the oxygen consumption quantity.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as limited by the foregoing description and is only limited by the scope of the appended claims.

[Industrial Applicability]

According to the present invention, even in a case in which there is a possibility that leakage hydrogen originating from purges and the like is contained in exhaust gas from a hydrogen reactor such as a fuel cell or the like, it is possible to accurately calculate a total quantity of hydrogen consumption of the hydrogen reactor without having to provide a separate hydrogen concentration sensor or modify the vehicle.

**Claims**

1. A hydrogen consumption quantity measurement system that measures a hydrogen consumption quantity in a test body, which is formed by a moving body or portion thereof that includes a hydrogen reactor that causes hydrogen to undergo a chemical reaction, and that utilizes energy obtained from this chemical reaction, comprising:

   an oxygen concentration sensor that measures a concentration of oxygen contained in exhaust gas from the test body;
   an oxidation catalyst that is disposed between the hydrogen reactor and the oxygen concentration sensor and that oxidizes hydrogen contained in the exhaust gas using oxygen contained in that exhaust gas; and
   a hydrogen consumption quantity calculation unit that, based on the oxygen concentration measured by the oxygen concentration sensor, calculates a hydrogen consumption quantity.

2. The hydrogen consumption quantity measurement system according to Claim 1, wherein

   the exhaust gas from the test body is introduced in either a diluted or undiluted state into the oxygen concentration sensor as a measurement gas, and
   the hydrogen consumption quantity measurement system is further provided with a flow meter that measures a flow rate of the exhaust gas either after dilution or before dilution, and
   the hydrogen consumption quantity calculation unit calculates a consumption quantity of hydrogen that has reacted with oxygen based on a difference between a concentration of oxygen in the measurement gas measured by the oxygen concentration sensor and a concentration of oxygen in a reference gas which is being used for comparison, and on the flow rate of exhaust gas measured by the flow meter.

3. The hydrogen consumption quantity measurement system according to Claims 1 or 2, further comprising a dehumidifier that is disposed between the oxidation catalyst and the oxygen concentration sensor, and that removes moisture contained in the measurement gas.

4. The hydrogen consumption quantity measurement system according to Claim 3, further comprising a moisture meter that measures a concentration of moisture in the measurement gas introduced into the oxygen concentration sensor, wherein moisture partial pressure correction is performed based on the moisture concentration measured by the moisture meter.

5. The hydrogen consumption quantity measurement system according to any one of Claims 1 through 4, further comprising a flow meter that measures a flow rate of exhaust gas discharged from the test body.

6. The hydrogen consumption quantity measurement system according to any one of Claims 1 through 5, wherein the hydrogen reactor is a fuel cell or a hydrogen engine.

7. The hydrogen consumption quantity measurement system according to any one of Claims 1 through 6, wherein the oxygen concentration sensor is a paramagnetic oxygen sensor.

8. The hydrogen consumption quantity measurement system according to Claims 1 through 7, wherein another oxygen concentration sensor is provided in parallel with the oxygen concentration sensor, and
   the oxidation catalyst oxidizes the exhaust gas introduced into either one of the two oxygen concentration sensors.

9. The hydrogen consumption quantity measurement system according to Claim 8, wherein moisture meters are provided that measure respective moisture concentrations of the measurement gas introduced into the two oxygen concentration sensors, and moisture partial pressure correction is performed based on the moisture concentrations measured by these moisture meters.

10. The hydrogen consumption quantity measurement system according to Claims 1 through 9, further comprising:

    a measurement gas flow path that guides the exhaust gas to the oxygen concentration sensor by causing it to pass through the oxidation catalyst;
    a bypass flow path that introduces the exhaust gas into the oxygen concentration sensor without causing it to pass through the oxidation catalyst; and
    a switching mechanism that switches the flow path that is connected to the oxygen concentration sensor between the measurement gas flow path and the bypass flow path.

11. The hydrogen consumption quantity measurement system according to any one of Claims 1 through 10, further comprising:

    a dilution mechanism that dilutes the exhaust gas using a dilution gas and then supplies the diluted gas to the oxygen concentration sensor; and
    a measurement gas flow path that introduces measurement gas diluted by the dilution gas to the oxygen sensor.

12. The hydrogen consumption quantity measurement system according to any one of Claims 1 through 11, further comprising:

    a dilution mechanism that dilutes the exhaust gas using a dilution gas and then supplies the diluted gas to the oxygen concentration sensor; and
    a reference gas flow path that introduces the dilution gas to the oxygen concentration sensor as a reference gas.

13. The hydrogen consumption quantity measurement system according to Claim 12, wherein

    the dilution mechanism is equipped with a sampling bag in which the dilution gas is collected, and
    the reference gas flow path introduces the reference gas from the sampling bag into the oxygen concentration sensor.

14. The hydrogen consumption quantity measurement system according to any one of Claims 1 through 10, further comprising a third oxygen sensor that is used to measure air, wherein the hydrogen consumption quantity measurement system is a vehicle on-board system.

15. A hydrogen leakage quantity measurement system that measures a hydrogen leakage quantity from a hydrogen reactor using an oxidation catalyst that is provided in the hydrogen consumption quantity measurement system according to Claim 1, and an oxygen concentration sensor, wherein

    the oxygen concentration sensor measures a concentration of oxygen in measurement gas that has passed through the oxidation catalyst, and a concentration of oxygen in measurement gas that has not passed through the oxidation catalyst, and
    there is provided a hydrogen leakage quantity calculation unit that calculates a hydrogen leakage quantity from a difference between the oxygen concentration in exhaust gas that has passed through the oxidation catalyst and been measured by the oxygen concentration sensor, and the oxygen concentration in exhaust gas that has not passed through the oxidation catalyst.

16. A hydrogen leakage quantity measurement system that measures a hydrogen leakage quantity from a hydrogen reactor using an oxidation catalyst that is provided in the hydrogen consumption quantity measurement system according to Claim 1, and an oxygen concentration sensor, wherein there are provided a dehumidifier that is disposed between the oxidation catalyst and the oxygen concentration sensor, and that removes moisture generated by the oxidation catalyst, and a hydrogen leakage quantity calculation unit that calculates a hydrogen leakage quantity from a difference between a moisture concentration before the oxidation catalyst and a moisture concentration after the oxidation catalyst.

17. The hydrogen leakage quantity measurement system according to Claims 15 or 16, further comprising a third oxygen sensor that is used to measure air, wherein the hydrogen leakage quantity measurement system is a vehicle on-board system.

18. A calibration system that calibrates an oxygen concentration sensor provided in the hydrogen consumption quantity measurement system according to Claim 1, wherein there is provided a hydrogenation mechanism that adds a predetermined quantity of hydrogen to the exhaust gas before the exhaust gas flows to the oxidation catalyst.

19. A hydrogen consumption quantity measurement method in which a hydrogen consumption quantity is measured in a test body, which is formed by a moving body or portion thereof that includes a hydrogen reactor that causes hydrogen to undergo a chemical reaction, and that utilizes energy obtained from this chemical reaction, and which is **characterized in that**,
after hydrogen contained in exhaust gas from the test body has been oxidized by oxygen contained in the exhaust gas, the concentration of oxygen contained in the exhaust gas is measured.

20. A hydrogen leakage quantity measurement method in which an oxygen concentration in exhaust gas after hydrogen contained in the exhaust gas discharged from a test body, which is formed by a moving body or portion thereof that includes a hydrogen reactor that causes hydrogen to undergo a chemical reaction, and that utilizes energy obtained from this chemical reaction, has been made to pass through an oxidation catalyst that performs oxidation using oxygen contained in the exhaust gas, and an oxygen concentration in the exhaust gas that has not been made to pass through the oxidation catalyst are measured, and a quantity of hydrogen leakage from a fuel cell is measured by comparing these oxygen concentrations.

21. A calibration method in which an oxygen concentration sensor provided in the hydrogen consumption quantity measurement system according to Claim 1 is calibrated, and which is **characterized in that** a predetermined quantity of hydrogen is added to the exhaust gas before the exhaust gas flows to the oxidation catalyst.

OXYGEN CONSUMPTION
QUANTITY MEASUREMENT
SYSTEM

HYDROGEN
COMBUSTION
REACTOR

EXHAUST
GAS FLOW
PATH

OXIDATION
CATALYST

OXIDATION CONCENTRATION SENSOR

HYDROGEN
CONSUMPTION QUANTITY
CALCULATION UNIT

FIG.1

FIG.2

FIG.3

**FIG.4**

FIG.5

FIG. 6

**FIG.7**

EP 4 317 933 A1

FIG.8

## INTERNATIONAL SEARCH REPORT

| | |
|---|---|
| International application No. | |
| **PCT/JP2022/016943** | |

**A. CLASSIFICATION OF SUBJECT MATTER**

*G01M 3/20*(2006.01)i; *H01M 8/00*(2016.01)i; *H01M 8/04*(2016.01)i; *H01M 8/04313*(2016.01)i; *H01M 8/0438*(2016.01)i; *H01M 8/0444*(2016.01)i; *H01M 8/04492*(2016.01)i; *H01M 8/10*(2016.01)i; *H01M 8/12*(2016.01)i; *G01M 17/007*(2006.01)i
FI: H01M8/04 Z; H01M8/0444; H01M8/0438; H01M8/04492; H01M8/10 101; H01M8/12 101; H01M8/00 Z; H01M8/04313; H01M8/04 J; H01M8/04 H; G01M17/007 Z; G01M3/20 L

According to International Patent Classification (IPC) or to both national classification and IPC

**B. FIELDS SEARCHED**

Minimum documentation searched (classification system followed by classification symbols)

G01M3/20; H01M8/00; H01M8/04; H01M8/04313; H01M8/0438; H01M8/0444; H01M8/04492; H01M8/10; H01M8/12; G01M17/007

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-123139 A (NISSAN MOTOR CO., LTD.) 12 May 2005 (2005-05-12) paragraphs [0008]-[0070], fig. 3, 8, 10 | 1, 3-4, 6-7, 11, 14, 19 |
| Y | | 2, 5 |
| A | | 8-10, 12-13, 15-18, 20-21 |
| Y | JP 2018-29038 A (MITSUBISHI HITACHI POWER SYSTEMS, LTD.) 22 February 2018 (2018-02-22) paragraph [0077] | 2, 5 |
| A | JP 2002-352824 A (NISSAN MOTOR CO., LTD.) 06 December 2002 (2002-12-06) | 1-21 |
| A | JP 2002-289237 A (TOYOTA MOTOR CORP.) 04 October 2002 (2002-10-04) | 1-21 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 June 2022** | **21 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2022/016943**

| Patent document cited in search report | | | Publication date (day/month/year) | Patent family member(s) | | | Publication date (day/month/year) |
|---|---|---|---|---|---|---|---|
| JP | 2005-123139 | A | 12 May 2005 | (Family: none) | | | |
| JP | 2018-29038 | A | 22 February 2018 | (Family: none) | | | |
| JP | 2002-352824 | A | 06 December 2002 | (Family: none) | | | |
| JP | 2002-289237 | A | 04 October 2002 | JP | 2009-277670 | A | |
| | | | | US | 2002/0094469 | A1 | |
| | | | | US | 2008/0070090 | A1 | |
| | | | | US | 2010/0151360 | A1 | |
| | | | | DE | 10201668 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MASARU YANO ; TAKUYA SHIMOSAKA ; SHIGEYUKI ISHIDOYA.** Development of a Fuel Consumption Measuring Method without Vehicle Modification - Oxygen Balance Method using a Constant Volume Sampler. *JARI Research Journal,* 01 April 2020

- **MASARU YANO ; EIJI KURODA.** Development of Fuel-Consumption Measuring Method for FCV without Vehicle Modification - Experiment Verification of Oxygen-Balance Method. *JARI Research Journal,* July 2008, vol. 30 (7